# EUROPÄISCHE PATENTANMELDUNG

(11) **EP 3 574 934 A1**
(43) Veröffentlichungstag der Anmeldung: **04.12.2019**
(21) Anmeldenummer: 18201263.3
(22) Anmeldetag: 18.10.2018
(51) Int. Cl.: A61M 1/10

(54) **FLUIDPUMPE**

(30) Priorität: 28.05.2018 EP 18174614
(71) Anmelder: Berlin Heart GmbH, 12247 Berlin (DE)
(72) Erfinder: Richert, Hendryk, 12487 Berlin (DE); FOSTER, Graham, Sketty, Swansea, SA2 8BH (GB)
(74) Vertreter: Pfenning, Meinig & Partner mbB

(57) **Zusammenfassung**

Die vorliegende Erfindung betrifft eine Fluidpumpe zum Fördern eines Fluids, insbesondere von Blut, umfassend: ein Gehäuse mit einem Fluideinlass und einem Fluidauslass, einen Rotor, welcher in dem Gehäuse um eine Rotationsachse drehbar angeordnet ist, und einen Rotorkörper und mindestens ein mit dem Rotorkörper starr verbundenes Förderelement zur Förderung des Fluids vom Fluideinlass zum Fluidauslass aufweist, wobei der Rotor in dem Gehäuse radial zur Rotationsachse mittels eines passiven Magnetlagers sowie axial und radial mittels eines einlassseitig oder auslassseitig angeordneten mechanischen und/oder hydrodynamischen Lagers gelagert ist. Die erfindungsgemäße Fluidpumpe zeichnet sich durch ein auf einer dem mechanischen und/oder hydrodynamischen Lager gegenüberliegenden Seite des Rotors angeordnetes Sicherheitslager mit einer ersten starr mit dem Rotor verbundenen Sicherheitslagerkomponente und einer zweiten starr mit dem Gehäuse verbundenen Sicherheitslagerkomponente aus, wobei während eines Betriebs der Fluidpumpe ein axialer und radialer Abstand zwischen der ersten und der zweiten Sicherheitslagerkomponente größer ist als ein radialer Minimalabstand zwischen dem Rotor und dem Gehäuse, und wobei das Sicherheitslager eingerichtet ist, eine Auslenkung des Rotors in radialer Richtung innerhalb des Gehäuses zu beschränken.

## Beschreibung

Die Erfindung betrifft eine Fluidpumpe zum Fördern eines Fluids, insbesondere von Blut.

Im Stand der Technik sind Blutpumpen mit hydrodynamischen, magnetischen und mechanischen Lagersystemen sowie Kombinationen davon bekannt. Weiterhin sind Hybridlagersysteme mit einem passiv magnetischen Lager und einem mechanischen Lager bekannt. Insbesondere werden dabei zwei magnetische Radiallager mit einem axialen Freiheitsgrad eingesetzt, der durch zwei mechanische (Auf-) Lager festgelegt wird.

Eine weitere beliebte Form von Blutpumpen verfügt über zwei mechanische Lager, die als sphärische, konische oder kegelige Axiallager ausgeführt sein können und den Nachteil haben, dass es praktisch nicht möglich ist, die axialen Lagerspalte einerseits so einzustellen, dass sie klein genug sind, damit kein Blut oder nur ein bestimmter Anteil des Blutes in die Lager eindringen kann.

Ferner bleiben die Lagerspalte während eines Betriebs der Blutpumpe nicht konstant, sondern werden sich durch Abnutzung verändern. Dringt Blut in die Lagerspalte ein, wird es dort starken Scherkräften unterworfen und durch beispielsweise Hämolyse, Plättchenaktivierung und andere negative Effekte geschädigt bzw. zerstört. Andererseits können die Lagerspalte in diesen mechanischen Lagern auch nicht so vergrößert werden, dass Scherkräfte reduziert werden, da die Vergrößerung des Axialspaltes zu Instabilitäten beim Betrieb der Pumpe führt.

Vollständig aktiv magnetisch gelagerte Pumpen haben den Nachteil, dass sie groß und komplex aufgebaut sind.

Die Aufgabe der vorliegenden Erfindung ist es daher, eine Fluidpumpe zum Fördern eines Fluids, insbesondere von Blut, bereitzustellen, die klein und einfach aufgebaut ist, die Blutschädigung minimiert und eine hohe Effizienz aufweist.

Die erfindungsgemäße Fluidpumpe zum Fördern eines Fluids, insbesondere von Blut, umfasst ein Gehäuse mit einem Fluideinlass und einem Fluidauslass, einen Rotor, welcher in dem Gehäuse um eine Rotationsachse drehbar angeordnet ist, und einen Rotorkörper und mindestens ein mit dem Rotorkörper starr verbundenes Förderelement zur Förderung des Fluids vom Fluideinlass zum Fluidauslass, wobei der Rotor in dem Gehäuse radial zur Rotationsachse mittels eines passiven Magnetlagers sowie axial und radial mittels eines einlassseitig oder auslassseitig angeordneten mechanischen und/oder hydrodynamischen Lagers gelagert ist.

Unter einem mechanischen und/oder hydrodynamischen Lager werden zum einen rein mechanische Lager oder rein hydrodynamische Lager sowie zum anderen Lager verstanden, welche sowohl mechanische als auch hydrodynamische Lagermerkmale aufweisen. Beispielsweise kann das mechanische und/oder hydrodynamische Lager als mechanisches Ball-Cup-Lager ausgebildet sein, wobei zwischen zwei Lagerkomponenten des Ball-Cup-Lagers ein Fluid-Film vorhanden ist, der zusätzlich einen hydrodynamischen Beitrag zur Lagerung liefert.

Die erfindungsgemäße Fluidpumpe zeichnet sich dadurch aus, dass auf einer dem mechanischen und/oder hydrodynamischen Lager gegenüberliegenden Seite des Rotors ein Sicherheitslager angeordnet ist, welches eine erste starr mit dem Rotor verbundene Sicherheitslagerkomponente und eine zweite starr mit dem Gehäuse verbundene Sicherheitslagerkomponente aufweist, wobei während eines Betriebs der Fluidpumpe ein axialer und radialer Abstand zwischen der ersten und der zweiten Sicherheitslagerkomponente größer ist als ein radialer Minimalabstand zwischen dem Rotor und dem Gehäuse, und wobei das Sicherheitslager eingerichtet ist, eine Auslenkung des Rotors in radialer Richtung innerhalb des Gehäuses zu beschränken.

Bei der vorliegenden erfindungsgemäßen Fluidpumpe werden alle Freiheitsgrade bereits durch das axial und radial wirkende mechanische und/oder hydrodynamische Lager und das radial wirkende Magnetlager festgelegt. Mithilfe des Magnetlagers kann der Rotor zusätzlich entweder zum mechanischen und/oder hydrodynamischen Lager hin oder vom mechanischen und/oder hydrodynamischen Lager weg vorgespannt werden, um die Kraft im Lager bzw. den Lagerspalt zu vergrößern bzw. zu reduzieren. Das mechanische und/oder hydrodynamische Lager kann auf diese Weise mit einer definierten Kraft beaufschlagt werden, sodass dieses unter normalen Bedingungen stabil arbeitet. Durch die konstante Vorspannung des Magnetlagers (im Rahmen des über die Pumpenlebensdauer zu erwartenden Verschleißes im mechanischen Auflager) passt sich die axiale Position des Rotors dynamisch an jede Änderung der Lagergeometrie im mechanischen und/oder hydrodynamischen Lager an, sodass der Lagerspalt im mechanischen und/oder hydrodynamischen Lager während der Pumpenlebensdauer immer so optimiert wird, dass ein gewünschter Anteil des von der Fluidpumpe geförderten Fluids oder kein Fluid in das mechanische und/oder hydrodynamische Lager eindringen kann. Das Sicherheitslager dient weiterhin dazu ein axiales und/oder radiales Herausfallen des Rotors aus dem Lagersystem zu verhindern, falls große Störkräfte auf den Rotor wirken, die die Haltekraft des Magnetlagers überwinden könnten. Das Sicherheitslager kann den Rotor axial und radial abfangen und somit einen Kontakt von empfindlicheren Rotorbestandteilen, wie z.B. dem Förderelement, mit dem Gehäuse verhindern. Dadurch, dass das Sicherheitslager gegenüberliegend und damit weit weg vom mechanischen und/oder hydrodynamischen Lager angeordnet ist, kann das Lagerspiel im Sicherheitslager, also ein Abstand zwischen den Sicherheitslagerkomponenten, trotz eines geringen Laufspiels zwischen dem Förderelement und dem Gehäuse relativ groß gewählt werden. Dadurch kann zwar das durch die Fluidpumpe fließende Fluid in das Sicherheitslager eindringen, jedoch sind aufgrund des großen Lagerspiels die auf das Fluid wirkenden Scherkräfte gering. Das geringe Laufspiel zwischen dem Förderelement und dem Gehäuse erhöht die Effizienz der Fluidpumpe. Somit ermöglicht die erfindungsgemäße Fluidpumpe ein effizientes Fördern eines Fluids, ein Anschlagen des Rotors am Gehäuse zu verhindern und eine Gefahr der Fluidschädigung, im Falle von Blut einer Blutschädigung, zu verringern.

Als passives Magnetlager kann prinzipiell jede Art von abstoßendem Magnetlager verwendet werden. Insbesondere sind jedoch Magnetlager bestehend aus mehreren axial antiparallel magnetisierten Permanentmagneten als sogenannte Squeeze-field Anordnungen sowie Halbach-Anordnungen mit der Kombination aus axial und radial magnetisierten Magneten besonders geeignet für diese Anwendung.

In einer vorteilhaften Ausgestaltung der Erfindung kann das Magnetlager in axialer Richtung auf einer zum Sicherheitslager hin gelegenen Seite des Rotorkörpers angeordnet sein. Auf diese Weise ist der Abstand zu einem Drehpunkt des mechanischen und/oder hydrodynamischen Lagers groß und es ist weniger Kraft notwendig, um den Rotor in seiner Mittellage bezüglich der radialen Richtung zwischen den Lagern zu halten.

Das Magnetlager kann mindestens einen ersten und einen zweiten Magneten aufweisen, wobei der erste Magnet in dem Gehäuse angeordnet ist und der zweite Magnet in dem Rotorkörper angeordnet ist, wobei der zweite Magnet gegenüber dem ersten Magneten in axialer Richtung zum mechanischen und/oder hydrodynamischen Lager hin derart versetzt angeordnet sein kann, dass der Rotorkörper in axialer Richtung zum mechanischen und/oder hydrodynamischen Lager hin und/oder gegen einen Fluidstrom gerichtet vorgespannt wird.

Das mechanische und/oder hydrodynamische Lager kann eine erste starr mit dem Rotor verbundene Lagerkomponente und eine zweite starr mit dem Gehäuse verbundene Lagerkomponente aufweisen, und das Magnetlager kann derart konfigurierbar sein, dass während eines Betriebs der Fluidpumpe ein Abstand zwischen der ersten und der zweiten Lagerkomponente minimiert wird und somit geringer ist als der axiale und/oder radiale Abstand zwischen der ersten Sicherheitslagerkomponente und der zweiten Sicherheitslagerkomponente.

Das Sicherheitslager kann weiterhin insbesondere derart ausgestaltet sein, dass während eines Betriebs der Fluidpumpe zwischen der ersten und der zweiten Sicherheitslagerkomponente ein axialer Abstand von ≥ 10 µm und/oder ≤ 500 µm, insbesondere von ≥ 50 µm und/oder ≤ 150 und ein radialer Abstand von ≥ 50 µm und/oder ≤ 1000 µm, insbesondere von ≥ 150 µm und/oder ≤ 500 µm beträgt. Der axiale und radiale Abstand weisen eine Größe auf, bei der auf das in den Lagerspalt des Sicherheitslagers eindringende Fluid geringere Scherkräfte wirken als bei Lagerspalten in mechanischen und/oder hydrodynamischen Lagern, die als Hauptlager dienen. Ferner befinden sich der axiale und radiale Lagerspalt nahe bzw. auf der Rotationsachse, wo geringe tangentiale Geschwindigkeiten und daher geringe Scherkräfte herrschen. Somit stellt das Sicherheitslager einerseits einen definierten und sicheren Anschlag für den Rotor dar, ohne andererseits eine Quelle für eine Schädigung des Fluids zu sein.

Weiterhin ist es besonders bevorzugt, wenn das Sicherheitslager derart in der Fluidpumpe angeordnet ist, dass ein Abstand zwischen dem mechanischen und/oder hydrodynamischen Lager und dem Sicherheitslager maximal ist, insbesondere mehr als doppelt so groß ist wie ein Abstand zwischen dem mechanischen und/oder hydrodynamischen Lager und dem Förderelement. In diesem Fall ist das Sicherheitslager weit weg vom mechanischen und/oder hydrodynamischen Lager und somit weit weg vom Drehpunkt des mechanischen und/oder hydrodynamischen Lagers, um welchen der Rotor durch äußere Störkräfte, wie z.B. einer g-Kraft (Kraft, die auf die Fluidpumpe bei Beschleunigung der Fluidpumpe wirkt), auslenkbar ist, wenn die Störkräfte die Haltekraft des Magnetlagers überwinden. Durch den großen Abstand von diesem Drehpunkt kann bei gleicher Winkeldifferenz der Rotationsachse zur Mittellage des Rotors im Sicherheitslager ein größerer radialer Abstand zwischen den Sicherheitslagerkomponenten vorgesehen werden als bei einem näher zum Drehpunkt hin angeordneten Sicherheitslager, was eine Verringerung von Scherkräften im Sicherheitslager bewirkt. Weiterhin kann auf diese Weise ein geringes Laufspiel zwischen einem nahe am mechanischen und/oder hydrodynamischen Lager angeordneten Förderelement und dem Gehäuse ermöglicht werden, was die Effizient der Fluidpumpe erhöht, da das Sicherheitslager einen ausreichenden Anschlagschutz bietet.

In einer weiteren vorteilhaften Ausgestaltung der Erfindung kann das Sicherheitslager auch außerhalb eines vom Fluideinlass zum Fluidauslass führenden primären Fluidweges, und somit außerhalb eines primären Fluidflusses, angeordnet sein, insbesondere kann das Sicherheitslager auf einer dem mechanischen und/oder hydrodynamischen Lager abgewandten Seite einer Volute des Gehäuses angeordnet sein. Insbesondere kann auch das magnetische Lager auf einer dem mechanischen und/oder hydrodynamischen Lager abgewandten Seite der Volute außerhalb des primären Fluidweges angeordnet sein. Der Vorteil dieser Anordnung des Magnetlagers besteht darin, dass das Magnetlager kleiner gestaltet werden kann, da Magnetlagerkomponenten des Magnetlagers dichter beieinander angeordnet werden können und so auch durch kleinere Magneten eine ausreichend große Magnetkraft erzeugbar ist, um den Rotor in der vorbestimmten Lage zu halten. Durch den vergrößerten Abstand zwischen magnetischem Lager und dem Drehpunkt des mechanischen und/oder hydrodynamischen Lagers kann das magnetische Lager weiterhin eine größere Hebelwirkung in radialer Richtung des mechanischen und/oder hydrodynamischen Lagers erzeugen und so den Rotor mit geringerem Kraftaufwand in der vorbestimmten Lage halten.

Es ist vorteilhaft, wenn das Förderelement in einem dem mechanischen und/oder hydrodynamischen Lager benachbarten Bereich des Rotorkörpers angeordnet ist. Hierdurch ist ein relativ freies Verkippen des Rotors aus seiner Mittellage bezüglich der radialen Richtung trotz eines geringen Abstands des Förderelements von dem Gehäuse möglich.

Besonders vorteilhaft ist es, wenn das Förderelement derart am Rotorkörper angeordnet ist, dass das Förderelement das mechanische und/oder hydrodynamische Lager radial zur Rotationsachse überlappt. In diesem Fall kann das Förderelement einerseits um den Drehpunkt des mechanischen und/oder hydrodynamischen Lagers herum angeordnet sein, was ein freies Verkippen des Rotors aus seiner Mittellage ohne ein Anstoßen am Gehäuse ermöglicht.

Andererseits kann das Förderelement im Verhältnis zum Rotorkörper groß ausgestaltet sein, was eine höhere Effizienz der Fluidpumpe ermöglicht.

Weiterhin kann es vorteilhaft sein, das Förderelement in einem Bereich des Rotorkörpers anzuordnen, in dem ein Abstand zwischen einer Oberfläche des Rotorkörpers und dem Gehäuse größer als ein Minimalabstand zwischen der Oberfläche und dem Gehäuse ist. Insbesondere kann das Förderelement in einer Volute des Gehäuses angeordnet sein. Hierdurch kann das Förderelement größer gestaltet werden, was die Effizienz der Fluidpumpe erhöht, und eine Gefahr des Anstoßens des Förderelements am Gehäuse verringert werden, da die Volute ein im Verhältnis zum übrigen Teil der Fluidpumpe großes freies Volumen aufweist.

Üblicherweise kann der Rotor eine Vielzahl von Förderelementen aufweisen. Insbesondere kann dann eine Teilmenge der Vielzahl von Förderelementen benachbart zum mechanischen und/oder hydrodynamischen Lager und eine weitere Teilmenge der Vielzahl von Förderelementen in einer Volute des Gehäuses angeordnet sein. Dies steigert die Effizienz der Fluidpumpe.

Besonders bevorzugt ist es, wenn das Förderelement eine Länge und/oder eine Breite von ≥ 2 mm und/oder ≤ 20 mm, insbesondere ≥ 5 mm und/oder ≤ 10 mm aufweist.

In einer weiteren vorteilhaften Ausgestaltung der Erfindung kann das Förderelement auf einer dem Gehäuse zugewandten Seite eine Axialschnittkontur aufweisen, welche an eine innere Axialschnittkontur des Gehäuses auf einer dem Förderelement zugewandten Seite des Gehäuses angepasst ist, sodass ein radialer Abstand zwischen dem Förderelement und dem Gehäuse entlang zumindest eines Bereichs des Förderelements, insbesondere zumindest über ≥ 40%, ≥ 50% oder ≥ 80% einer Länge des Förderelements in axialer Richtung, in axialer Richtung im Wesentlichen konstant ist. Dies erhöht insbesondere die Effizienz der Fluidpumpe und kann das freie Verkippen des Rotors begünstigen.

Ein relativ freies Verkippen des Rotors ist insbesondere dann möglich, wenn die Axialschnittkontur des Förderelements und die innere Axialschnittkontur des Gehäuses korrespondierende sphärische Krümmungen aufweisen, insbesondere in dem Fall, in dem das Förderelement benachbart zum oder um den Drehpunkt des mechanischen und/oder hydrodynamischen Lagers herum angeordnet ist.

Weiterhin ist es vorteilhaft, wenn das Sicherheitslager rotationssymmetrisch um die Rotationsachse des Rotors ausgebildet ist, wobei ein Maximaldurchmesser des Sicherheitslagers kleiner ist als ein Maximaldurchmesser des Rotors. Je näher sich der Lagerspalt des Sicherheitslagers an der Rotationsachse befindet, umso geringer ist eine Tangentialgeschwindigkeit des in das Sicherheitslager eindringenden Fluids. Hierdurch sind auch die von den Sicherheitslagerkomponenten auf das Fluid ausgeübten Scherkräfte geringer.

Das mechanische und/oder hydrodynamische Lager kann insbesondere ein sphärisches Auflager, ein Ball-Cup-Lager und/oder ein konisches Auflager sein.

Die Fluidpumpe kann eine Axialflusspumpe oder eine Radialflusspumpe oder eine Kombination aus Axialfluss- und Radialflusspumpe sein.

Die erste und/oder die zweite Sicherheitslagerkomponente und/oder die erste und/oder zweite Lagerkomponente des mechanischen und/oder hydrodynamischen Lagers können ein keramisches, polymeres, metallisches und/oder einkristallines Material aufweisen oder daraus bestehen.

Weiterhin können die erste und/oder die zweite Sicherheitslagerkomponente und/oder die erste und/oder zweite Lagerkomponente des mechanischen und/oder hydrodynamischen Lagers eine einen Verschleiß hemmende und/oder eine Blutschädigung vermindernde, insbesondere Hämolyse vermindernde, Beschichtung aufweisen. Die Beschichtung kann insbesondere Silikone, PU, MPC, Mehrfachzucker, allg. Polymere, Keramiken, diamantartige Stoffe, Metallschichten, Nitride, Oxide, Mehrfachschichten, bioaktive, medikamenthaltige und/oder andere Schichten aufweisen oder daraus bestehen.

Im Folgenden werden mehrere Ausführungsbeispiele einer erfindungsgemäßen Fluidpumpe anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des Beispiels und weiterer Merkmale des Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: ein erstes Ausführungsbeispiel einer erfindungsgemäßen Fluidpumpe in einer Axialschnittansicht,
- Figur 2: ein zweites Ausführungsbeispiel einer erfindungsgemäßen Fluidpumpe in einer Axialschnittansicht,
- Figur 3: ein drittes Ausführungsbeispiel einer erfindungsgemäßen Fluidpumpe in einer Axialschnittansicht,
- Figur 4: ein Schema, welches den Anschlagschutz durch das Sicherheitslager veranschaulicht,
- Figur 5: ein viertes Ausführungsbeispiel einer erfindungsgemäßen Fluidpumpe und
- Figur 6: ein fünftes Ausführungsbeispiel einer erfindungsgemäßen Fluidpumpe.

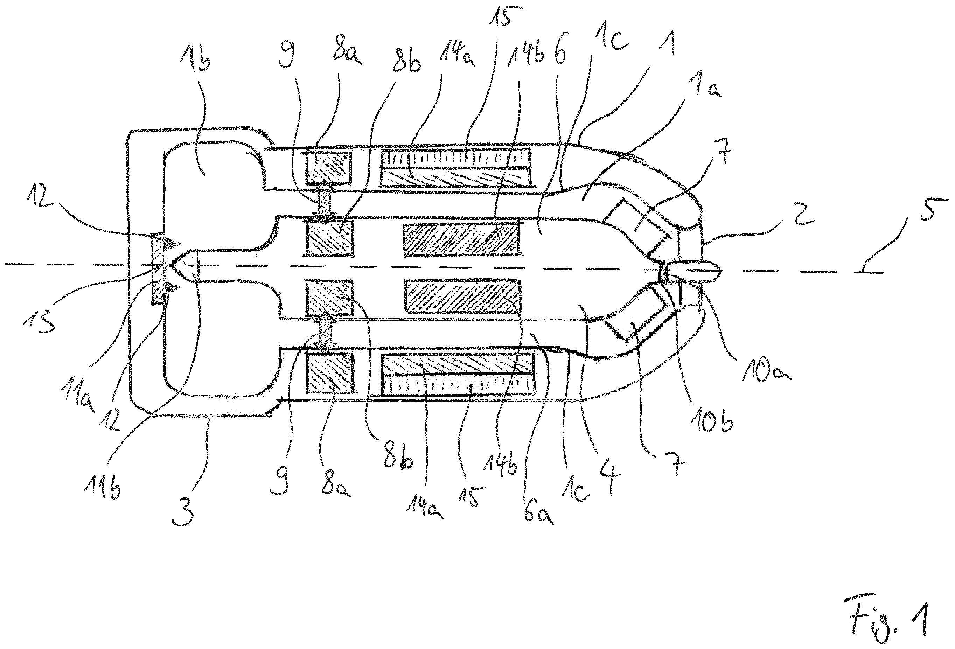

Figur 1 zeigt ein erstes Ausführungsbeispiel einer erfindungsgemäßen Fluidpumpe in einer Axialschnittansicht. Die Fluidpumpe weist ein Gehäuse 1 auf, welches eine im Wesentlichen hohlzylindrische äußere Form aufweist, die im Wesentlichen rotationssymmetrisch um eine Mittelachse 5 des Gehäuses 1 ist. Das Gehäuse 1 weist einen hohlzylinderförmig ausgestalteten Innenraum 1a auf, welcher von einer inneren Wandung 1b des Gehäuses 1 begrenzt wird. Das Gehäuse 1 weist weiterhin einen Fluideinlass 2 an einer Seite des Gehäuses 1 im Bereich der Mittelachse 5 auf. Auf der dem Fluideinlass 2 gegenüberliegenden Seite des Gehäuses 1 weist das Gehäuse 1 eine Volute 1b auf, welche als schneckenförmige Erweiterung des Gehäuses 1 ausgebildet ist. Durch das Gehäuse 1 ist ein Fluid vom Fluideinlass 2 entlang der Mittelachse 5 bis in die Volute 1b führbar. Innerhalb der Volute 1b wird das Fluid nicht mehr entlang der Mittelachse 5 sondern zunächst radial von der Mittelachse 5 weg und dann um die Mittelachse 5 herum zu einem in der Volute 1b angeordneten Fluidauslass 3 geführt (Der Fluidauslass 3 ist hier nicht direkt dargestellt, dass Bezugszeichen 3 deutet lediglich eine mögliche Position des Fluidauslasses an.) Der Fluidauslass 3 ist derart in der schneckenförmigen Volute 1b angeordnet, dass das Fluid durch den Fluidauslass 3 tangential zur Mittelachse 5 aus dem Gehäuse 1 austreten kann. In dem Gehäuse 1 ist weiterhin ein Rotor 4 mit einem Rotorkörper 6 angeordnet. Der Rotor 4 ist im Bereich des Fluideinlasses 2 mittels eines mechanischen und/oder hydrodynamischen Lagers 10a, 10b gelagert. Weiterhin weist die Fluidpumpe ein passives Magnetlager 8a, 8b auf, welches in einem zur Volute 1b hin gelegenen Bereich der Fluidpumpe angeordnet ist und einen Gehäusemagneten 8a und einen Rotormagneten 8b aufweist. Sowohl der Gehäusemagnet 8a als auch der Rotormagnet 8b sind zylinder- bzw. hohlzylinderförmig ausgestaltet und konzentrisch zueinander und um die Mittelachse 5 des Gehäuses 1 herum angeordnet. Das Magnetlager 8a, 8b hält den Rotor 5 auf der Mittelachse 5 des Gehäuses 1, sodass eine Rotationsachse des Rotors 4 mit der Mittelachse 5 des Gehäuses 1 zusammenfällt. Bei den Magneten des Magnetlagers 8a, 8b handelt es sich um abstoßende Magneten, die den Rotor auf der Mittelachse 5 durch eine in radialer Richtung wirkende abstoßende Magnetkraft 9 halten. Das mechanische und/oder hydrodynamische Lager ist als ein sphärisches Auflager ausgestaltet und weist eine starr mit dem Gehäuse 1 verbundene erste Lagerkomponente 10a und eine starr mit dem Rotor 4 verbundene zweite Lagerkomponente 10b auf. Die Lagerkomponente 10a ist in einem zur Lagerkomponente 10b gelegenen Bereich kugelförmig oder allgemein konvex ausgebildet, während die Lagerkomponente 10b in einem zur Lagerkomponente 10a gelegenen Bereich kalottenförmig oder allgemein konkav ausgebildet ist. Es ist dabei unerheblich, auf welcher Seite die konvexe Form liegt. Unter konvex oder konkav fallen auch nicht stetige Formen wie z. B. Kegelstümpfe oder abgestufte Lager. Somit schränkt das mechanische und/oder hydrodynamische Lager 10a, 10b eine translatorische Bewegung des Rotors 4 in axialer Richtung in Richtung des Fluideinlasses 2 und in radialer Richtung ein. Das Magnetlager 8a, 8b ist so konfigurierbar, dass der Rotor 4 in das mechanische und/oder hydrodynamische Lager 10a, 10b während eines Betriebs der Fluidpumpe hineingedrückt werden kann oder entgegen den auftretenden Fluidkräften im Betrieb das mechanische und/oder hydrodynamische Lager entlastet. Bei der Auslegung des Magnetlagers 8a, 8b sollte daher eine potentielle Vorspannung des Rotors 4 gegen einen Fluidfluss durch die Fluidpumpe mit eingerechnet werden. Durch die Schubkraft des Magnetlagers 8a, 8b auf den Rotor 4, welche groß genug ist den Rotor während des Betriebs der Fluidpumpe stets in das mechanische und/oder hydrodynamische Lager zu drücken, wird vermieden, dass sich während des Betriebs der Fluidpumpe, beispielsweise durch Verschleiß, der Lagerspalt zwischen den Lagerkomponenten 10a und 10b vergrößert. Der Lagerspalt zwischen den Lagerkomponenten 10a, 10b wird somit durch das Magnetlager 8a, 8b stets minimiert, wodurch verhindert wird, dass größere Bestandteile des Fluids, welche beispielsweise im Falle von Blut Erythrozyten oder andere Zellen oder Proteine sein können, zwischen die Lagerkomponenten 10a, 10b gelangt und dort geschädigt oder zerstört werden. Auf einer dem mechanischen und/oder hydrodynamischen Lager gegenüberliegenden Seite der Fluidpumpe im Bereich des Fluidauslasses 3 ist in der Volute 1b ein Sicherheitslager 11a, 11b mit einer starr mit dem Gehäuse 1 verbundenen ersten Sicherheitslagerkomponente 11a und einer starr mit dem Rotor 4 verbundenen zweiten Sicherheitslagerkomponente 11b angeordnet. Während eines normalen Betriebs der Fluidpumpe findet kein Kontakt zwischen der Sicherheitslagerkomponenten 11a und der Sicherheitslagerkomponente 11b statt. Der Rotor 4 kann sich vielmehr innerhalb des Sicherheitslagers 11a, 11b frei bewegen. Treten nun äußere Störkräfte, wie z.B. unter einer Beschleunigung der Fluidpumpe, auf, kann es zu einer starken Auslenkung des Rotors 4 um das mechanische und/oder hydrodynamische Lager 10a, 10b kommen. Das mechanische und/oder hydrodynamische Lager 10a, 10b stellt einen Drehpunkt für den Rotor 4 dar. Um diese Auslenkung zu begrenzen, weist die Sicherheitslagerkomponente 11a ein hohlzylinderförmiges (konkaves), radiales Begrenzungselement 12 auf, welches einen radialen Anschlag für die Lagerkomponente 11b darstellt, wobei auch hier die Seite für die konkave Sicherheitslagerkomponente irrelevant ist. Weiterhin weist die Sicherheitslagerkomponente 11a einen axialen Anschlag 13 auf, welcher verhindert, dass der Rotor 4 vollständig aus dem mechanischen und/oder hydrodynamischen Lager 10a, 10b in axialer Richtung in Richtung der Volute 1b herausfällt. Da es sich bei dem Sicherheitslager 11a, 11b um ein kontaktloses Sicherheitslager handelt, kann ein Lagerspalt im Sicherheitslager, also ein Abstand zwischen der ersten und der zweiten Sicherheitslagerkomponente 11a, 11b, so groß gewählt werden, dass zwar Fluid zwischen die Sicherheitslagerkomponenten 11a und 11b gelangen kann, jedoch auch gleichzeitig die in dem Sicherheitslager 11a, 11b herrschenden Scherkräfte auf das Fluid gering sind. Somit ermöglicht das Sicherheitslager 11a, 11b einerseits eine Sicherung gegen ein Anschlagen von empfindlichen Bestandteilen des Rotors 4 bei gleichzeitig geringer Fluidschädigung durch Scherkräfte. Weiterhin ist es im Gegensatz zu den Fluidpumpen des Standes der Technik bei der vorliegenden erfindungsgemäßen Fluidpumpe nicht notwendig den Lagerspalt im Sicherheitslager 11a, 11b exakt einzustellen bzw. stets zu minimieren, um ein Eindringen von Fluid in den Lagerspalt zu verhindern, da ein Kontakt mit dem Sicherheitslager 11a, 11b das Fluid nicht schädigt. Weiterhin können das radiale Begrenzungselement 12 und der axiale Anschlag 13 des Sicherheitslagers 11a, 11b getrennt sein und der Rotor 4 kurz vor der Volute 1b durch herausstehende Elemente, die auch eine Förderwirkung entwickeln können, radial begrenzt werden. Denkbar sind hier Streben, Schaufelelemente, Ringe oder Ringsegmente. Weiterhin ist die erfindungsgemäße Fluidpumpe einfach aufbaubar und miniaturisierbar, da lediglich ein passives Magnetlager eingesetzt wird.

Der Rotor 4 weist außerdem benachbart zum Fluideinlass 2 Förderelemente 7 auf, welche das Fluid nach Eintritt durch den Fluideinlass 2 entlang der Mittelachse 5 in Richtung des Fluidauslasses 3 befördern. Die äußere, zum Gehäuse 1 zugewandte Kontur der Förderelemente 7 ist derart an die Kontur der inneren Wandung 1c des Gehäuses 1 angepasst, dass sich ein geringer Abstand (Laufspiel des Förderelements 7) zwischen dem Förderelement 7 und der inneren Wandung 1c ergibt. Durch ein geringes Laufspiel des Förderelements 7 wird verhindert, dass das Fluid an dem Förderelement 7 vorbei fließt bzw. nicht von dem Förderelement 7 erreicht und in Richtung des Fluidauslasses 3 beschleunigt wird. Ein geringes Laufspiel zwischen Förderelement 7 und Gehäuse 1 erhöht somit die Effizienz der Fluidpumpe. Weiterhin ist benachbart zum Fluideinlass 2 die innere Wandung 1c um das Förderelement 7 herum sphärisch gestaltet, sodass in diesem Bereich der Rotor 4 über einen möglichst großen Winkelbereich frei um den Drehpunkt des mechanischen und/oder hydrodynamischen Lagers 10a, 10b verkippen kann. Hierdurch wird ein frühes Anschlagen der Förderelemente 7 am Gehäuse 1 verhindert, und ein Anschlagen des Rotors 4 erfolgt erst an dem Begrenzungselement 12 des Sicherheitslagers 11a, 11b.

Der Rotor 4 wird mittels eines im Gehäuse 1 angeordneten Motorstators 14a, 15 und eines im Rotorkörper 6 angeordneten Motormagneten 14b angetrieben. Der Motorstator 14a, 15 und der Motormagnet 14b sind konzentrisch zueinander angeordnet.

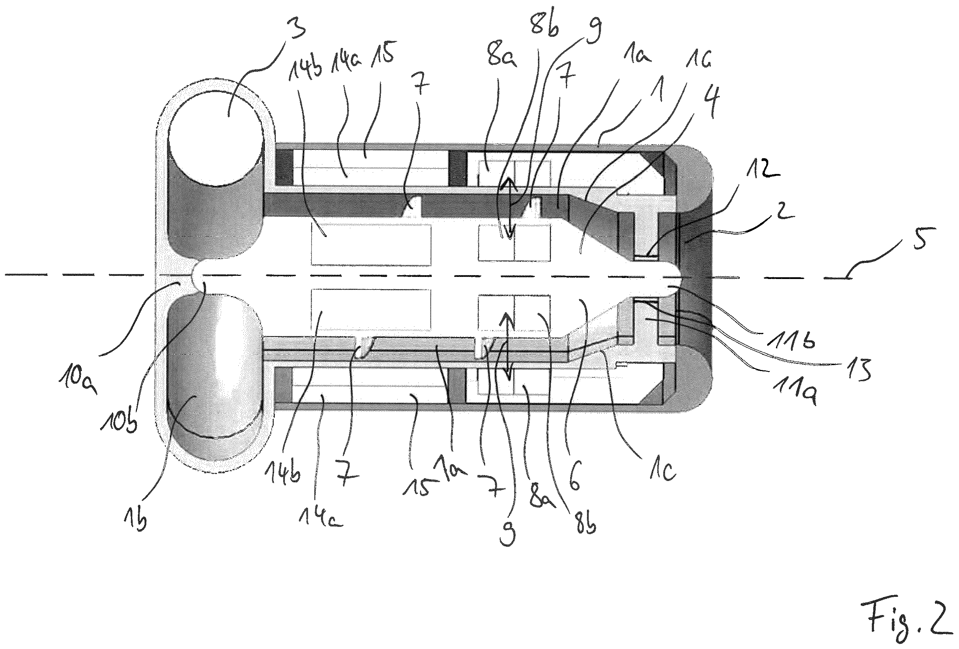

Figur 2 zeigt ein weiteres Ausführungsbeispiel einer erfindungsgemäßen Fluidpumpe in einer Axialschnittansicht. Im Gegensatz zum ersten Ausführungsbeispiel der Figur 1 ist bei der Fluidpumpe in Figur 2 das mechanische und/oder hydrodynamische Lager 10a, 10b in der Volute 1b und das Sicherheitslager 11a, 11b im Bereich des Fluideinlasses 2 angeordnet. Dementsprechend ist das radiale Magnetlager 8a, 8b in einem vorderen, also einem zum Fluideinlass 2 hin gelegenen Bereich der Fluidpumpe angeordnet, um eine größere Hebelwirkung auf den sich um den sich in der Volute 1b befindlichen Drehpunkt des mechanischen und/oder hydrodynamischen Lagers 10a, 10b verkippbaren Rotor 4 entfalten zu können. Das Magnetlager 8a, 8b ist weiterhin in diesem Ausführungsbeispiel so konfiguriert, dass der Rotor 4 während eines Betriebs der Fluidpumpe stets in das mechanische und/oder hydrodynamische Lager hineingedrückt wird, um einen Lagerspalt zwischen den Lagerkomponenten 10a und 10b zu minimieren. Im Gegensatz zum ersten Ausführungsbeispiel wird in dem zweiten Ausführungsbeispiel der Rotor 4 somit nicht zum Fluideinlass 2, sondern in die Volute 1c zum Fluidauslass 3 gedrückt. Je nachdem wie stark die zum Fluidauslass 3 gerichtete Vorspannung des Rotors 4 gegen den Fluidstrom mittels des Magnetlagers 8a, 8b eingestellt ist, tritt in der Fluidpumpe gemäß dem zweiten Ausführungsbeispiel während des Betriebs eine Entlastung des mechanischen und/oder hydrodynamischen Lagers 10a, 10b auf. Wie auch beim ersten Ausführungsbeispiel findet beim zweiten Ausführungsbeispiel im normalen Betrieb der Fluidpumpe kein Kontakt zwischen den Sicherheitslagerkomponenten 11a und 11b statt. Das Sicherheitslager 11a, 11b fängt den Rotor 4 mittels der radialen und axialen Begrenzungselemente 12 und 13 lediglich bei einer starken Auslenkung des Rotors 4 aus seiner Ideallage auf der Mittelachse 5 aufgrund äußerer Störkräfte ab.

Ein weiterer Unterschied zum ersten Ausführungsbeispiel besteht in der Anordnung der Förderelemente 7. Diese befinden sich im mittleren Bereich des hohlzylinderförmigen Innenraums 1a und sind so gestaltet, dass sie das Fluid in axialer Richtung parallel zur Mittelachse beschleunigen. Aufgrund der Anordnung der Förderelemente 7 im mittleren Bereich des Innenraums 1a können die Förderelemente 7 bei einer starken Auslenkung des Rotors 4 aus seiner Ideallage früher an die innere Wandung 1c anstoßen als dies im ersten Ausführungsbeispiel in Figur 1 der Fall ist. Daher ist auch der radiale Lagerspalt zwischen der zweiten Sicherheitslagerkomponente 11b und dem radialen Begrenzungselement 12 kleiner als in Figur 1 ausgebildet, um ein Anschlagen der Förderelemente 7 an der inneren Wandung 1c zu vermeiden. Der radiale Abstand zwischen der Sicherheitslagerkomponente 11b und dem Begrenzungselement 12 ist jedoch auch in Figur 2 größer als das Laufspiel der Förderelemente 7 zur inneren Wandung 1c.

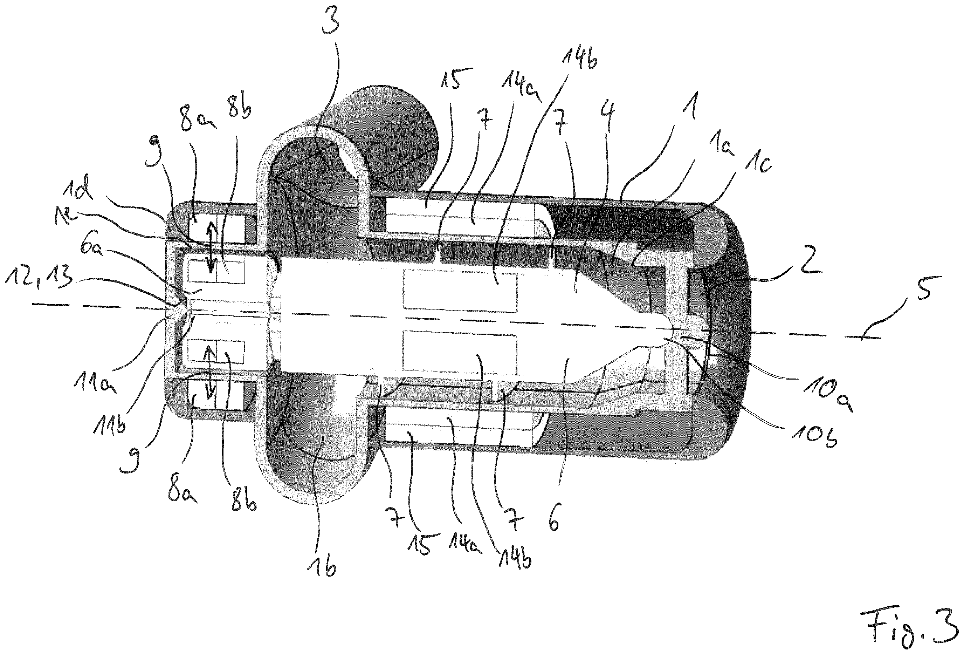

Figur 3 zeigt ein drittes Ausführungsbeispiel einer erfindungsgemäßen Fluidpumpe in einer Axialschnittansicht. Im dritten Ausführungsbeispiel ist wie im ersten Ausführungsbeispiel das mechanische und/oder hydrodynamische Lager 10a, 10b im Bereich des Fluideinlasses 2 angeordnet. Anders als im ersten Ausführungsbeispiel ist das Sicherheitslager 11a, 11b nun auf einer dem Fluideinlass 2 abgewandten Seite der Volute 1b außerhalb eines vom Fluideinlass 2 zum Fluidauslass 3 führenden primären Fluidweges angeordnet. Hierzu weisen auf einer dem Fluideinlass 2 abgewandten Seite der Volute 1b sowohl das Gehäuse 1 eine ebenfalls hohlzylinderförmige Gehäuseerweiterung 1d als auch der Rotorkörper 6 eine Rotorkörpererweiterung 6a auf. Die mit der Gehäuseerweiterung 1d starr verbundene erste Sicherheitslagerkomponente 11a ist dornförmig ausgebildet und greift in eine als sich in Richtung der Volute 1b verjüngende Bohrung ausgebildete zweite Sicherheitslagerkomponente 11b in der Rotorkörpererweiterung 6a ein und begrenzt somit eine translatorische Bewegung des Rotors 4 in axialer Richtung und eine Drehbewegung des Rotors 4 um das mechanische und/oder hydrodynamische Lager 10a, 10b.

Auch das Magnetlager 8a, 8b ist im zweiten Ausführungsbeispiel auf der dem Fluideinlass 2 abgewandten Seite der Volute 1b angeordnet, wobei sich der Gehäusemagnet 8a in der Gehäuseerweiterung 1d und der Rotormagnet 8b in der Rotorkörpererweiterung 6a befindet. Aufgrund des großen axialen Abstandes zum mechanischen und/oder hydrodynamischen Lager 10a, 10b durch die Lage außerhalb der Volute 1b kann das Magnetlager 8a, 8b eine größere Hebelwirkung auf den Rotor 4 zur radialen Fixierung des Rotors 4 ausüben. Ein weiterer Vorteil der Fluidpumpe gemäß dem dritten Ausführungsbeispiel besteht insbesondere darin, dass ein kleineres magnetisches Lager einsetzbar ist, da es möglich ist, den Gehäusemagneten 8a und den Rotormagneten 8b geringer voneinander beabstandet anzuordnen und auf diese Weise die magnetische Kraft zu erhöhen. Ein geringerer Abstand zwischen dem Gehäusemagneten 8a und dem Rotormagneten 8b ist möglich, da zwischen der inneren Wandung 1e der Gehäuseerweiterung 1d und der Rotorkörpererweiterung 6a ein kleinerer Abstand als im Bereich des primären Fluidweges ermöglicht werden kann, da keine Förderelemente untergebracht werden müssen.

Die Förderelemente 7 sind im dritten Ausführungsbeispiel wie im zweiten Ausführungsbeispiel im mittleren Bereich des Innenraums 1a angeordnet. Jedoch ist in Figur 3 der axiale Abstand zum mechanischen und/oder hydrodynamischen Lager 10a, 10b so groß, dass der radiale Lagerspalt im Sicherheitslager 11a, 11b größer als in Figur 2 ausgebildet sein kann, ohne dass die Förderelemente 7 vor einem Kontakt der Sicherheitslagerkomponenten 11a, 11b an die innere Wandung 1c des Gehäuses 1 anschlagen.

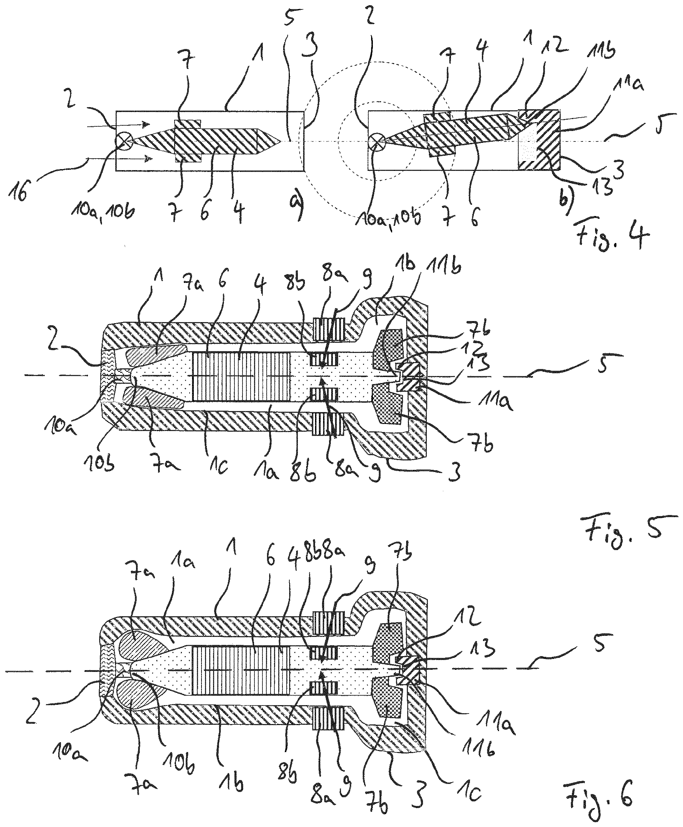

Figur 4 zeigt ein Schema, welches einen Anschlagschutz durch das Sicherheitslager 11a, 11b veranschaulicht. In Figur 4a) befindet sich der Rotor 4 in seiner Ideallage, das heißt, die Rotationsachse des Rotors 4 und die Mittelachse 5 fallen zusammen. In Figur 4b) ist der Rotor 4 bezüglich der Mittelachse 5 verkippt. Das mechanische und/oder hydrodynamische Lager 10a, 10b ist im Bereich des Fluideinlasses 2, durch welchen ein Fluidstrom 16 in die Fluidpumpe eintritt, angeordnet und stellt für den Rotor 4 einen Drehpunkt dar. Das Verkippen des Rotors 4 bezüglich der Mittelachse 5 wird durch den Lagerspalt im Sicherheitslager 11a, 11b, die Lage der Förderelemente 7 und die Rückstellkraft des magnetischen Lagers begrenzt. Ein Anschlagen der Förderelemente 7 ist unbedingt zu vermeiden, da es hierbei schnell zu großen Schäden in der Fluidpumpe kommt, die sich negativ auf den Patienten auswirken. Als Störkräfte und Störmomente können insbesondere radiale Fluidkräfte, Kipp-Momente durch Fluidkräfte und radiale Kräfte und Kipp-Momente durch g-Kräfte sowie gyroskopisch induzierte Kippmomente auftreten. Den stärksten quasistationären Anteil liefert dabei die g-Kraft.

Eine möglichst freie Kippung des Rotors 4 ohne ein Anstoßen der Förderelemente 7 am Gehäuse 1 kann beispielsweise durch eine spezielle Geometrie der Förderelemente 7 erreicht werden. Der zur Verfügung stehende Kippwinkel des Rotors 4 wird geometrisch durch das Laufspiel der Förderelemente 7 und den Abstand zum Lagerpunkt des mechanischen und/oder hydrodynamischen Lagers 10a, 10b definiert. Dieser Kippwinkel wird maximiert, wenn die Förderelemente 7 möglichst nah am Lagerpunkt bzw. sphärisch um diesen herum angeordnet werden. Mit einer sphärischen Außenkontur versehene Förderelemente 7a, wie beispielsweise in Figur 6 dargestellt, kann somit die Kippproblematik größtenteils vermeiden. Das Laufspiel ist in diesem Fall vollständig unabhängig vom Kippwinkel des Rotors 4 und das unkontrollierte Anschlagen des Rotors 4 kann mit einem einfachen Sicherheitslager 11a, 11b verhindert werden. Die notwendige Lagersteifigkeit des Magnetlagers 8a, 8b der Fluidpumpe wird dann ausschließlich durch externe Kräfte und Momente im Zusammenspiel mit dem zur Verfügung stehenden Kippwinkel des Rotors 4 definiert.

Figur 5 zeigt ein viertes Ausführungsbeispiel einer erfindungsgemäßen Fluidpumpe in einer Axialschnittansicht. Das mechanische und/oder hydrodynamische Lager 10a, 10b ist wie in Figuren 1 und 3 im Bereich des Fluideinlasses 2 angeordnet. Die Förderelemente 7 sind direkt benachbart zu dem mechanischen und/oder hydrodynamischen Lager 10a, 10b angeordnet und überlappen dieses teilweise. Weiterhin ist die der inneren Wandung 1c zugewandte Außenkontur der Förderelemente 7a über im Wesentlichen die gesamte axiale Länge der Förderelemente 7a an einen Verlauf der inneren Wandung 1c angepasst, sodass sich über den gesamten Verlauf der axialen Länge der Förderelemente 7a ein geringes Laufspiel zur inneren Wandung 1c ergibt. Durch die Überlappung der Förderelemente 7a mit dem mechanischen und/oder hydrodynamischen Lager 10a, 10b können die Förderelemente 7a größer gestaltet werden und den Fluidfluss früher am Fluideinlass 2 aufnehmen und beschleunigen. Die dargestellten Förderelemente 7a können daher das Fluid sehr effizient durch das Gehäuse 1 pumpen. Weiterhin können die das mechanische und/oder hydrodynamische Lager 10a, 10b überlappenden Förderelemente 7a das mechanische und/oder hydrodynamische Lager 10a, 10b umspülen und so die Funktionsfähigkeit des Lagers 10a, 10b unterstützen. Zusätzlich verläuft die innere Wandung 1c in einem spitzen Winkel zur Mittelachse 5, der sich vom Fluideinlass 2 aus öffnet. Dies verhindert trotz des geringen Laufspiels ein frühes Anschlagen der Förderelemente 7a an der inneren Wandung 1c. Das in der Volute 1b angeordnete Sicherheitslager 11a, 11b weist dabei einen Lagerspalt auf, der einerseits ein Anschlagen der Förderelemente 7a an der Wandung 1c verhindert und andererseits groß genug ist um Scherkräfte im Lager 11a, 11b gering zu halten und so eine Fluidschädigung zu verhindern.

Anders als in den vorangegangenen Ausführungsbeispielen weisen die Fluidpumpen des vierten und fünften Ausführungsbeispiels alternative oder zusätzliche Förderelemente 7b in der Volute 1b auf. Diese dienen dazu das Fluid schneller aus der Fluidpumpe tangential durch den Fluidauslass 3 heraus zu befördern und erhöhen dadurch nochmals die Effizienz der Fluidpumpe, bzw. stellen als alleinige Schaufeln eine reine Radialkonfiguration dar, bei der generell die Abstände zwischen Schaufeln und Gehäuse größer ausgeführt werden können als bei den weiter oben beschriebenen axialen oder halbaxialen Pumpenkonzepten.

Figur 6 zeigt ein fünftes Ausführungsbeispiel einer erfindungsgemäßen Fluidpumpe in einer Axialschnittansicht. Die Fluidpumpe des fünften Ausführungsbeispiels umfasst eine Kombination von Förderelementen 7a und Einlassgeometrie, die bereits in der Beschreibung der Figur 4 erwähnt wurde. Das fünfte Ausführungsbeispiel der Figur 6 unterscheidet sich vom vierten Ausführungsbeispiel der Figur 5 darin, dass die innere Wandung 1c um das mechanische und/oder hydrodynamische Lager 10a, 10b herum sphärisch gestaltet ist. Dies ermöglicht dem Rotor 4 einen großen Kippwinkel um das mechanische und/oder hydrodynamische Lager 10a, 10b. Die Förderelemente 7a sind auch hier in ihrer zur inneren Wandung 1c zugewandten Kontur an den Verlauf der inneren Wandung 1c angepasst, sodass sich über einen großen Anteil der axialen Länge der Förderelemente 7a ein geringes Laufspiel ergibt. Aufgrund des sphärischen Verlaufs der inneren Wandung 1c wird ein frühes Anschlagen der Förderelemente 7a trotz des geringen Laufspiels vermieden. Ein Anschlag erfolgt dann erst im Sicherheitslager 11a, 11b. Weiterhin sind auch im fünften Ausführungsbeispiel der Figur 6 wie bereits im vierten Ausführungsbeispiel der Figur 5 alternative oder zusätzliche Förderelemente 7b in der Volute 1b angeordnet, welche es ermöglichen, das Fluid schneller aus der Fluidpumpe durch den Fluidauslass 3 heraus zu befördern.

## Patentansprüche

1. Fluidpumpe zum Fördern eines Fluids, insbesondere von Blut, umfassend:
ein Gehäuse mit einem Fluideinlass und einem Fluidauslass,
einen Rotor, welcher in dem Gehäuse um eine Rotationsachse drehbar angeordnet ist, und
einen Rotorkörper und mindestens ein mit dem Rotorkörper starr verbundenes Förderelement zur Förderung des Fluids vom Fluideinlass zum Fluidauslass aufweist,
wobei der Rotor in dem Gehäuse radial zur Rotationsachse mittels eines passiven Magnetlagers sowie axial und radial mittels eines einlassseitig oder auslassseitig angeordneten mechanischen und/oder hydrodynamischen Lagers gelagert ist,
**gekennzeichnet durch**
ein auf einer dem mechanischen und/oder hydrodynamischen Lager gegenüberliegenden Seite des Rotors angeordnetes Sicherheitslager mit einer ersten starr mit dem Rotor verbundenen Sicherheitslagerkomponente und einer zweiten starr mit dem Gehäuse verbundenen Sicherheitslagerkomponente, wobei während eines Betriebs der Fluidpumpe ein axialer und radialer Abstand zwischen der ersten und der zweiten Sicherheitslagerkomponente größer ist als ein radialer Minimalabstand zwischen dem Rotor und dem Gehäuse, und wobei das Sicherheitslager eingerichtet ist, eine Auslenkung des Rotors in radialer Richtung innerhalb des Gehäuses zu beschränken.

2. Fluidpumpe nach dem vorhergehenden Anspruch, **dadurch gekennzeichnet, dass** das Sicherheitslager derart ausgestaltet ist, dass während eines Betriebs der Fluidpumpe zwischen der ersten und der zweiten Sicherheitslagerkomponente ein axialer Abstand ≥ 10 µm und/oder ≤ 500 µm und ein radialer Abstand ≥ 50 µm und/oder ≤ 1000 µm beträgt.

3. Fluidpumpe nach einem der vorhergehenden Ansprüche, **dadurch gekennzeichnet, dass** das Sicherheitslager derart in der Fluidpumpe angeordnet ist, dass ein Abstand zwischen dem mechanischen und/oder hydrodynamischen Lager und dem Sicherheitslager maximal ist, insbesondere mehr als doppelt so groß ist wie ein Abstand zwischen dem mechanischen und/oder hydrodynamischen Lager und dem Förderelement.

4. Fluidpumpe nach einem der vorhergehenden Ansprüche, **dadurch gekennzeichnet, dass** das Sicherheitslager außerhalb eines vom Fluideinlass zum Fluidauslass führenden primären Fluidweges angeordnet ist, insbesondere, dass das Sicherheitslager auf einer dem mechanischen und/oder hydrodynamischen Lager abgewandten Seite einer Volute des Gehäuses angeordnet ist.

5. Fluidpumpe nach einem der vorhergehenden Ansprüche, **dadurch gekennzeichnet, dass** das Förderelement in einem dem mechanischen und/oder hydrodynamischen Lager benachbarten Bereich des Rotorkörpers angeordnet ist.

6. Fluidpumpe nach dem vorhergehenden Anspruch, **dadurch gekennzeichnet, dass** das Förderelement derart am Rotorkörper angeordnet ist, dass das Förderelement das mechanische und/oder hydrodynamische Lager radial zur Rotationsachse überlappt.

7. Fluidpumpe nach einem der vorhergehenden Ansprüche, **dadurch gekennzeichnet, dass** das Förderelement in einem Bereich des Rotorkörpers angeordnet ist, in dem ein Abstand zwischen einer Oberfläche des Rotorkörpers und dem Gehäuse größer als ein Minimalabstand zwischen der Oberfläche und dem Gehäuse ist, insbesondere, dass das Förderelement in einer Volute des Gehäuses angeordnet ist.

8. Fluidpumpe nach einem der vorhergehenden Ansprüche, **dadurch gekennzeichnet, dass** das Förderelement auf einer dem Gehäuse zugewandten Seite eine Axialschnittkontur aufweist, welche an eine innere Axialschnittkontur des Gehäuses auf einer dem Förderelement zugewandten Seite des Gehäuses angepasst ist, sodass ein radialer Abstand zwischen dem Förderelement und dem Gehäuse entlang zumindest eines Bereichs des Förderelements, insbesondere zumindest über ≥ 40%, ≥ 50% oder ≥ 80% einer Länge des Förderelements in axialer Richtung, in axialer Richtung im Wesentlichen konstant ist.

9. Fluidpumpe nach dem vorhergehenden Anspruch, **dadurch gekennzeichnet, dass** die Axialschnittkontur des Förderelements und die innere Axialschnittkontur des Gehäuses korrespondierende sphärische Krümmungen aufweisen.

10. Fluidpumpe nach einem der vorhergehenden Ansprüche, **dadurch gekennzeichnet, dass** das Magnetlager in axialer Richtung auf einer zum Sicherheitslager hin gelegenen Seite des Rotorkörpers angeordnet ist.

11. Fluidpumpe nach einem der vorhergehenden Ansprüche, **dadurch gekennzeichnet, dass** das Magnetlager mindestens einen ersten und einen zweiten Magneten aufweist, wobei der erste Magnet in dem Gehäuse angeordnet ist und der zweite Magnet in dem Rotorkörper angeordnet ist, wobei der zweite Magnet gegenüber dem ersten Magneten in axialer Richtung zum mechanischen und/oder hydrodynamischen Lager hin versetzt ist, sodass der Rotorkörper in axialer Richtung gegen einen Fluidstrom vorgespannt ist.

12. Fluidpumpe nach dem vorhergehenden Anspruch, **dadurch gekennzeichnet, dass** das mechanische und/oder hydrodynamischen Lager eine erste starr mit dem Rotor verbundene Lagerkomponente und eine zweite starr mit dem Gehäuse verbundene Lagerkomponente aufweist, und das Magnetlager derart konfigurierbar ist, dass während des Betriebs der Fluidpumpe ein Abstand zwischen der ersten und der zweiten Lagerkomponente minimiert wird, insbesondere geringer ist als der axiale und/oder radiale Abstand zwischen der ersten Sicherheitslagerkomponente und der zweiten Sicherheitslagerkomponente.

13. Fluidpumpe nach einem der vorhergehenden Ansprüche, **dadurch gekennzeichnet, dass** die Fluidpumpe eine Axialflusspumpe oder eine Radialflusspumpe oder eine Kombination aus Axialfluss- und Radialflusspumpe ist.

14. Fluidpumpe nach einem der vorhergehenden Ansprüche, **dadurch gekennzeichnet, dass** die erste und/oder die zweite Sicherheitslagerkomponente und/oder dass die erste und/oder zweite Lagerkomponente ein keramisches, polymeres, metallisches und/oder einkristallines Material aufweisen oder daraus bestehen.

15. Fluidpumpe nach einem der vorhergehenden Ansprüche, **dadurch gekennzeichnet, dass** die erste und/oder die zweite Sicherheitslagerkomponente und/oder dass die erste und/oder zweite Lagerkomponente eine Verschleiß hemmende und/oder eine Blutschädigung vermindernde, insbesondere Hämolyse vermindernde, Beschichtung aufweist.
